# EUROPEAN PATENT SPECIFICATION

(11) **EP 3 414 317 B1**
(45) Date of publication and mention of the grant of the patent: **17.06.2026**
(21) Application number: 17713071.3
(22) Date of filing: 09.02.2017
(51) Int. Cl.: C12N 1/20, A61K 35/747, A23L 33/135, C12R 1/225

(54) **USE OF BACTERIAL STRAINS BELONGING TO THE SPECIES LACTOBACILLUS KEFIRI IN PAEDIATRICS TO GENERATE AND/OR MAINTAIN A STATE OF HOMEOSTASIS**
VERWENDUNG VON BAKTERIENSTÄMME DER GATTUNG LACTOBACILLUS KEFIRI IN DER PÄDIATRIE ZUR ERZEUGUNG UND/ODER STABILISIERUNG EINES HOMÖOSTASESTATUS
UTILISATION DE SOUCHES BACTÉRIENNES APPARTENANT À L'ESPÈCE LACTOBACILLUS KEFIRI EN PÉDIATRIE POUR GÉNÉRER ET/OU MAINTENIR UN ÉTAT D'HOMÉOSTASIE

(30) Priority: 12.02.2016 IT UB20160708; 10.05.2016 IT UA20163324
(43) Date of publication of application: 19.12.2018
(73) Proprietor: Probiotical S.p.A., 28100 Novara (IT); Hulka S.r.l., 45100 Rovigo (IT)
(72) Inventor: MOGNA, Giovanni, 28100 Novara (IT)
(74) Representative: Benedetto, Marco
(86) International application number: PCT/IB2017/050713
(87) International publication number: WO 2017/137920

(56) References cited:
- WO-A1-2012/001440
- CN-A- 104 560 793
- TW-A- 201 231 057
- US-A1- 2011 123 640
- P. CARASI ET AL: "Impact of Kefir Derived Lactobacillus kefiri on the Mucosal Immune Response and Gut Microbiota", JOURNAL OF IMMUNOLOGY RESEARCH, vol. 137, no. 3, 1 January 2015 (2015-01-01), US, pages 781 - 12, XP055324639, ISSN: 2314-8861, DOI: 10.1155/2015/361604
- ANONYMOUS: "Kefibios LKef fermenti lattici flacone con contagocce 6ml e 1 capsula separata", 12 June 2011 (2011-06-12), XP002764320, Retrieved from the Internet <URL:http://www.amicafarmacia.com/kefibios-lkef-fermenti-lattici-flacone-con-contagocce-6ml-e-1-capsula-separata.html> [retrieved on 20161116]
- DRAGO LORENZO ET AL: "Antibiotic susceptibility profile of a new Lactobacillus kefiri strain.", JOURNAL OF GLOBAL ANTIMICROBIAL RESISTANCE MAR 2016, vol. 4, 10 November 2015 (2015-11-10), pages 74 - 75, XP002770517, ISSN: 2213-7173

## Description

The present invention relates to the use of bacterial strains belonging to the species *Lactobacillus kefiri* in a method for the preventive or curative treatment of inflammatory intestinal diseases selected from the group comprising or, alternatively, consisting of: irritable bowel syndrome (IBS), inflammatory bowel disease (IBD), Crohn's disease, colics, colics in infants, colitis, and ulcerative colitis (UC). The present disclosure further relates to bacterial strains belonging to the species *Lactobacillus kefiri* having a high capacity to adhere to the intestinal epithelium; in particular, the present disclosure relates to the bacterial strains *Lactobacillus kefiri* LKF01 DSM 32079 LKEF and *Lactobacillus kefiri* LKF02 DSM 32080 for use in paediatrics. Finally, the present invention relates to a food composition, or a composition for a supplement, or a composition for a medical device, or a pharmaceutical composition (briefly all together the composition(s) of the present disclosure) comprising excipients and/or substances of a food or pharmaceutical grade formulation, and a mixture which comprises or, alternatively, consists of a bacterial strain selected from the group consisting of the bacterial strains belonging to the species *Lactobacillus kefiri,* for use in the preventive and/or curative treatment of inflammatory intestinal diseases such as irritable bowel syndrome (IBS), inflammatory bowel disease (IBD), Crohn's disease and ulcerative colitis (UC), in adult subjects and also in a chronic stage.

It is well known that the interactions between the bacteria inhabiting the intestinal epithelium and the cells of the immune system together play a fundamental role in maintaining the homeostasis of the intestine and the body in general. Bacterial recognition, through specific receptors, induces the expression and release of important immunological mediators, among which one may mention, by way of example, pro- and antiinflammatory chemokines and cytokines, which contribute to orchestrating both innate and adaptive immune responses.

DRAGO LORENZO ET AL: "Antibiotic susceptibility profile of a new Lactobacillus kefiri strain", JOURNAL OF GLOBAL ANTIMICROBIAL RESISTANCE MAR 2016, vol. 4, 10 November 2015, pages 74-75, XP002770517, ISSN: 2213-7173, discloses the bacteria strain *L. kefiri* LKF0L (DSM32079), its resistance to some common antibiotics and, thus, the possibility to use this bacterial strain in co-administration with antibiotics.

TW201231057A discloses that *L. Kefiri* M2 strain as an immunomodulatory strain which has anti-allergic and therapeutic effects on intestinal inflammation, and can be used for making anti-allergic drugs and improving allergy symptoms.

P. Carasi ET AL: "Impact of Kefir Derived Lactobacillus kefiri on the Mucosal Immune Response and Gut Microbiota", Journal of Immunology Research, vol. 137, no. 3, 1 January 2015, pages 781-12, discloses the use of *L. Kefiri* DIDCA 8348 strain in mice and concludes that it is a good candidate for use in gut inflammatory diseases.

There thus still continues to be keen interest in the scientific community and, at the same time, a highly felt need, on the part of those operating in the field, to identify, select and characterise resident bacterial strains (with a high adhesion capacity) capable of modulating the immune response at both the level of the intestinal mucosa and at a systemic level.

The above-mentioned interest is due to the fact that there is an increasingly strong demand for the development of functional health products having marked properties beneficial for the body and which can be used in the treatment (preventive or curative) of many inflammatory intestinal diseases.

By virtue of an intense, prolonged activity of research and development, the Applicant has succeeded in providing an adequate response to the aforementioned needs.

The present invention relates to bacterial strains belonging to the species *Lactobacillus kefiri,* identified as *Lactobacillus kefiri* LKF01 DSM 32079 and deposited by Probiotical SpA on 10.07.2015 with the institute DSMZ - Germany, and *Lactobacillus kefiri* LKF02 DSM 32080 deposited by Probiotical SpA on 10.07.2015 with the institute DSMZ - Germany, for use in the preventive or curative treatment of inflammatory intestinal diseases selected from the group comprising or, alternatively, consisting of: irritable bowel syndrome (IBS), inflammatory bowel disease (IBD), Crohn's disease, colics, colics in infants, colitis, ulcerative colitis (UC), preferably in adult subjects, and preferably also in a chronic stage.

The present invention relates to the use of bacterial strains belonging to the species *Lactobacillus kefiri* as defined above the preventive and/or curative treatment of inflammatory intestinal diseases, such as irritable bowel syndrome (IBS), inflammatory bowel disease (IBD), Crohn's disease and ulcerative colitis (UC), in adult subjects, also in a chronic stage, or in pediatrics, and having the features disclosed in the appended claims. The present invention relates to bacterial strains belonging to the species *Lactobacillus kefiri,* for use in the preventive and/or curative treatment of inflammatory intestinal diseases, such as irritable bowel syndrome (IBS), inflammatory bowel disease (IBD), Crohn's disease and ulcerative colitis (UC), in adult subjects, also in a chronic stage, or in pediatrics, and having the features disclosed in the appended claims.

The present invention further relates to a food composition, or a composition for a supplement, or a composition for a medical device, or a pharmaceutical composition (briefly all together the composition(s) of the present disclosure) comprising excipients and/or substances of a food or pharmaceutical grade formulation, and a mixture which comprises or, alternatively, consists of a bacterial strain selected from the group consisting of the bacterial strains belonging to the species Lactobacillus kefiri, for use in the preventive and/or curative treatment of inflammatory intestinal diseases, such as irritable bowel syndrome (IBS), inflammatory bowel disease (IBD), Crohn's disease and ulcerative colitis (UC), in adult subjects, also in a chronic stage, and having the features disclosed in the appended claims.

A preferred embodiment of the present invention relates to the bacterial strains LKF01 DSM 32079 LKEF and *Lactobacillus kefiri* LKF02 DSM 32080, for use in the preventive and/or curative treatment of inflammatory intestinal diseases, such as irritable bowel syndrome (IBS), inflammatory bowel disease (IBD), Crohn's disease and ulcerative colitis (UC), in adult subjects, also in a chronic stage, and having the features disclosed in the appended claims.

Other preferred embodiments of the disclosure are disclosed below in the detailed description that follows.
Figure 1 shows an apparatus for evaluating trans-epithelial electrical resistance on cell tissues.
Figure 2 shows the percentage of adhesion of some bacterial strains of the present study vis-a-vis CACO-2 cells.
Figure 3 shows the percentage of membrane integrity assessed relative to the basal TEER value of non-stimulated CACO-2 cells (basal value equal to 100%; pro-inflammatory stimulus equal to 40% and reading at 24 hours).
Figure 4 shows the so-called fold increase (FI) in membrane integrity assessed relative to the TEER value of CACO-2 cells stimulated with pro-inflammatory factors (TEER value of the CACO-2 stimulated with proinflammatory factors equal to 1; reading at 24 hours).
Figure 5 (A and B) shows the inverse relationship between the incidence of infectious diseases (A) and the incidence of immune diseases (B) from 1950 to 2000.

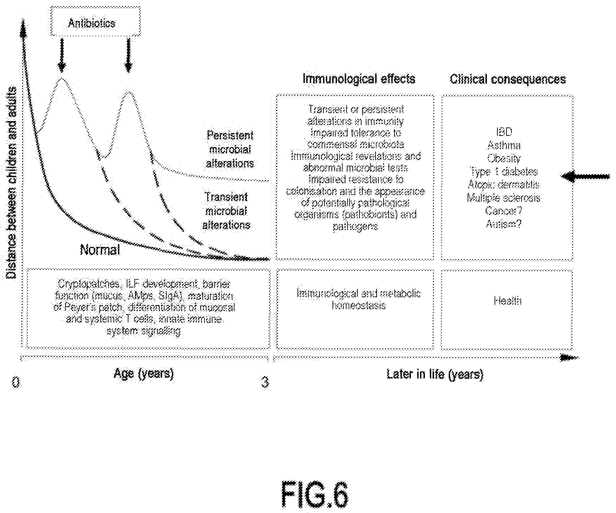

Figure 6 shows the distance between children and adults in the alterations of the transient and persistent microbial flora in children up to 3 years of age, namely, cryptopatches, ILF development, barrier function (mucus, AMps, SlgA), maturation of Peyer's patch, differentiation of mucosal and systemic T cells, and innate immune system signalling following antibiotic treatments. Furthermore, in children over three years of age and in adults it shows the direct immunological effects, namely, transient or persistent alterations in immunity, impaired tolerance to commensal microbiota, immunological revelations or abnormal microbial tests, impaired resistance to colonisation and the appearance of potentially pathological organisms (pathobionts) and pathogens and the clinical consequences, e.g. IBD, asthma, obesity, type 1 diabetes, atopic dermatitis, multiple sclerosis, cancer and autism.
Figure 7 refers to the colonisation of the intestine (preprandial administration) before and then one month after the administration of the bacterial strain *L. kefiri* LKF01 (DSM 32079) LKEF.
Figure 8 refers to the colonisation of the intestine (postprandial administration) before and then one month after the administration of the bacterial strain *L. kefiri* LKF01 (DSM 32079) LKEF.
Figure 9 refers to accelerated stability tests on the bacterial strain *L. kefiri* LKF01 (DSM 32079) LKEF, non-reconstituted, at 37°C.
Figure 10 refers to accelerated stability tests on the bacterial strain *L. kefiri* LKF01 (DSM 32079) LKEF, reconstituted, at 25°C.
Figure 11 refers to accelerated stability tests on the bacterial strain *L. kefiri* LKF01 (DSM 32079) LKEF, reconstituted, at 25°C, 30°C and 33°C.
Among the bacteria belonging to the genus *Lactobacillus,* the Applicant selected the species *Lactobacillus kefiri,* which demonstrated to have a surprising adhesion capacity compared to other species of lactobacilli. Subsequently, following a selection conducted on a vast group of bacterial strains belonging to the species *Lactobacillus kefiri,* the Applicant identified, selected and characterised the following bacterial strains :
   (i) *Lactobacillus kefiri* LKF01 DSM 32079 deposited by Probiotical SpA on 10.07.2015 with the institute DSMZ - Germany.
   (ii) *Lactobacillus kefiri* LKF02 DSM 32080 deposited by Probiotical SpA on 10.07.2015 with the institute DSMZ - Germany.

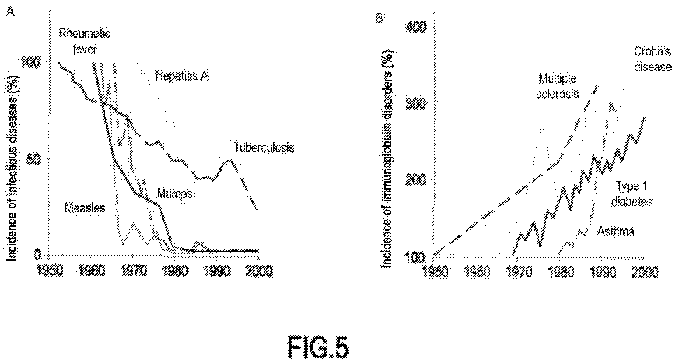

The bacterial strains belonging to the species *Lactobacillus kefiri* have shown to have a surprising property of adhering to the intestinal mucosa, which enables them to advantageously express their specific characteristics with a lasting, efficacious protective effect and optimal restoration of the integrity of the intestinal epithelium, such as to assure homeostasis. High adhesion contributes, on one hand, to regenerating the microbial flora which remains and lasts for a lifetime and, on the other hand, to improving protection and hence cell resistance; see Figures 5 and 6, and the scientific paper published in Nature Immunology 15, 307-310 (2014). From the above it may be inferred that it is fundamental to be able to intervene in human microbial flora, especially in the first three years of life, in such a way as to impart and develop healthy bacterial flora to assure a good state of homeostasis that will last over time.

Advantageously, the use of autochthonous resident probiotic bacteria - bacterial flora in the body (not transient), with a high adhesion capacity and capable of modulating the immune response both at the level of the intestinal mucosa and at a systemic level, such as those used in the present invention, represents an interesting solution, both in paediatrics in order to generate and/or maintain a state of homeostasis, and in the preventive and/or curative treatment of inflammatory intestinal diseases, such as irritable bowel syndrome (IBS), inflammatory bowel disease (IBD), Crohn's disease and ulcerative colitis (UC), in adult subjects, also in a chronic stage.

The present invention relates to a food composition, or a composition for a supplement, or a composition for a medical device, or a pharmaceutical composition (briefly all together the composition(s) of the present disclosure) comprising excipients and/or substances of a food or pharmaceutical grade formulation, and a mixture which comprises or, alternatively, consists of a bacterial strain selected from the group consisting of the bacterial strains belonging to the species Lactobacillus kefiri, for use in the preventive and/or curative treatment of inflammatory intestinal diseases, such as irritable bowel syndrome (IBS), inflammatory bowel disease (IBD), Crohn's disease and ulcerative colitis (UC), in adult subjects, also in a chronic stage.

Said bacterial strain in said composition belongs to the species *Lactobacillus kefiri* and is selected between the bacterial strain *Lactobacillus kefiri* LKF01 DSM 32079 deposited by Probiotical SpA on 10.07.2015 with the institute DSMZ - Germany, and the bacterial strain *Lactobacillus kefiri* LKF02 DSM 32080 deposited by Probiotical SpA on 10.07.2015 with the institute DSMZ - Germany. Preferably, said bacterial strains in said composition are in the form of bacterial cells in a coated form, with an animal or vegetable lipid coating. Alternatively, said bacterial strains in said composition are in the form of "bare", i.e. uncoated bacterial cells. Said bacterial strains/cells to be coated or microencapsulated can be in solid form, in particular in the form of a powder, granules, or a dehydrated or lyophilised powder.

The bacteria are coated or microencapsulated with a coating material comprising or, alternatively, consisting, of at least one lipid of animal or vegetable origin, using the techniques and processes known to the persons skilled in the art. Advantageously, the lipid is of vegetable origin.

The lipid coating is applied/formed with a multilayer or microencapsulation or multi-coating technique that entails the formation of a well-defined layer. The yield of the process for applying/forming the coating layer on the bacterial cells is greater than about 80%, but is usually greater than 90%.

The bacterial strains belonging to the species *L. kefiri* in lyophilised form can be coated or microencapsulated by means of a fluid bed technique (for example, top-spray or bottom-spray) in which the coating material, represented by lipids of vegetable origin, is applied externally on the bacteria after being heated and converted into a liquid state. The coated bacterial strains belonging to the species *L. kefiri* are then added, using known techniques, to the other components, excipients and process additives in order to yield the composition (food product, supplement product, medical device or pharmaceutical composition) of the present invention.

The coating material comprises or, alternatively, consists of at least one lipid of vegetable origin. The lipids are selected from the group comprising or, alternatively, consisting of saturated vegetable fats having a melting point comprised from 35 °C to 85 °C, preferably comprised from 45 to 70 °C. Advantageously, from 50 to 60 °C.

In a preferred embodiment, one can use saturated vegetable fats having a certain degree of hydrophilicity and/or hydrophobicity, which can be selected from group comprising mono- and di-glycerides of saturated fatty acids, polyglycerols esterified with saturated fatty acids and free saturated fatty acids.

The saturated fatty acids can be selected from the group comprising from 8 to 32 carbon atoms, preferably from 12 to 28 carbon atoms, even more preferably from 16 to 24 carbon atoms.

Advantageously, the lipid is selected from the group comprising or, alternatively, consisting of: (i) glyceryl dipalmitostearate E471, INCI (PCPC): glyceryl distearate, CAS: 85251-77-0 (or 1323-83-7), EINECS: 286-490-9 (or 215-359-0). An example of a commercial product is Biogapress Vegetal BM 297 ATO - Gattefosse SAS; (ii) polyglyceryl palmitostearate E475, I NCI: polyglyceryl-6-distearate, CAS: 61725-93-7. An example of a commercial product is Plurol Stearique WL 1009 -Gattefosse SAS.

The type and chemical nature of the lipid used in the coating layer depend on: (i) the chemical and physical properties of the finished product, (ii) the content of water or moisture or free water present in the finished product, for example a vegetable oil in which the coated bacteria are added, (iii) the ingredients, excipients and additives used to formulate the finished product or (iv) the physical state of the finished product.

In order to prepare a finished product (composition of the present invention) in powder or granular or lyophilised pharmaceutical form, for example in a sachet, capsule, tablet or stick, the lipid glyceryl dipalmitostearate E471 , INCI (PCPC): glyceryl distearate, CAS: 85251-77-0 (or 1323-83-7), EINECS: 286-490-9 (or 215-359-0) (Example of a commercial product, Biogapress Vegetal BM 297 ATO -Gattefosse SAS) is used to coat the bacterial strains belonging to the species *L. kefiri* preferably the bacterial strain *Lactobacillus kefiri* LKF01 DSM 32079 and the bacterial strain *Lactobacillus kefiri* LKF02 DSM 32080.

In order to prepare a finished product (composition of the present invention) in powder or granular or lyophilised form, containing the bacterial strains belonging to the species *L kefiri,* to be added to a vegetable oil so as to yield a pharmaceutical form, for example an oily suspension or an oil in drops, the lipid polyglyceryl palmitostearate E475, INCI: polyglyceryl-6-distearate, CAS: 61725-93-7 (example of a commercial product Plurol Stearique WL 1009 -Gattefosse SAS) is used to coat the bacterial strains belonging to the species *L. kefiri* they are preferably the bacterial strain *Lactobacillus kefiri* LKF01 DSM 32079 and the bacterial strain *Lactobacillus kefiri* LKF02 DSM 32080.

In the context of the present invention, "coating layer" means a coating layer applied externally to the surface of the bacterium/cell.

In one embodiment of the present invention, said composition comprises a vegetable oil selected from among an olive oil, a soybean oil, a corn oil, a rice oil or a sunflower oil, a maltodextrin and said mixture of strains which comprises or, alternatively, consists of *Lactobacillus kefiri* LKF01 DSM 32079 LKEF and *Lactobacillus kefiri* LKF02 DSM 32080, preferably in a ratio by weight comprised from 1:2 to 2:1 ; 1:0, 0:1, 1:1.

A preferred embodiment is represented by a bottle, preferably provided with a single-dose pipette, which comprises a container and a closure element consisting of a cap. The closure element has a housing indicated as an undercap or capsule, which serves to accommodate the bacterial strains of the present invention, with the bacterial cells in a coated or bare form.

The coated bacteria are accommodated in the undercap of said closure element. At the time of use, the undercap can be opened by rotating or vertically pushing the closure element and the coated bacteria contained therein fall into the liquid, preferably a vegetable oil, present inside the container. The bacteria can be mixed with said liquid simply by shaking to yield a suspension of bacterial strains in oil.

The container contains said vegetable oil, whereas the closure element comprises a capsule (isolated compartment) suitable for containing, in a manner such as to be isolated from the contents of the container, the bacterial strains of the present invention in lyophilised or powder or granular form. The capsule is opened at the time of use and the bacterial strains contained therein come into contact with said vegetable oil to yield the composition for use of the present invention.

Preferably, said bacterial strains, preferably in lyophilised form or in granules or in powder, are present at a concentration comprised from 1x10⁸ CFU/g a 1x10¹² CFU/g of bacteria, preferably at a concentration comprised from 1x10⁹CFU/g to 1x10¹¹ CFU/g of bacteria.

For example, a 7 ml bottle of vegetable oil with a dropper and separate capsule can contain about 300 mg of powder or lyophilisate of the bacterial strain *Lactobacillus kefiri* LKF01 DSM 32079 and/or bacterial strain *Lactobacillus kefiri* LKF02 DSM 32080 (preferably in a ratio by weight comprised from 1:2 to 2:1; 1:0, 0:1, 1:1) and a maltodextrin. The value per bottle (7 ml) is greater than or equal to 25x10⁹ CFU, whereas the value per dose (5 drops) is greater than or equal to 1x10⁹ CFU.

Preferably, said bacterial strains, preferably in lyophilised form or in granules or in powder, are present in said composition at a concentration comprised from 1x10⁸ CFU/g to 1x10¹² CFU/g of composition, preferably at a concentration comprised from 1x109 CFU/g to 1x1011 CFU/g of composition; even more preferably, said composition comprises a vegetable oil selected from among an olive oil, a soybean oil, a corn oil, a rice oil and a sunflower oil, a maltodextrin and said mixture of bacterial strains; preferably, 10 ml of oil comprise from 30x10⁹ CFU/g to 40x10⁹ CFU/g of composition.

Advantageously, the bacterial strains for use of the present invention belonging to the species Lactobacillus kefiri, i.e. the bacterial strains *Lactobacillus kefiri* LKF01 DSM 32079 and *Lactobacillus kefiri* LKF02 DSM 32080, are declared to be allergen free (free of all the potential allergens identified in Annex II of Regulation (EU) No. 1169/2011) because they are prepared according to the technology and production process patented by the company Probiotical SpA Novara (Italy) -WO2007/054989 A1 and EP 1869161 B1. Advantageously, the bacterial strains for use of the present invention belonging to the species *Lactobacillus kefiri,* i.e. the bacterial strains *Lactobacillus kefiri* LKF01 DSM 32079 and *Lactobacillus kefiri* LKF02 DSM 32080, can be used in combination with antibiotics without the risk of a transferable or transmittable antibiotic resistance (Drago L, et al. "Antibiotic susceptibility profile of a new L. kefiri strain"; J Global Antimicrob Resist (2015).

Alternatively, in the event of a concomitant antibiotic therapy, the bacterial strain *Lactobacillus kefiri* LKF01 DSM 32079 and/or bacterial strain *Lactobacillus kefiri* LKF02 DSM 32080 (or a composition for use of the present invention which contains said strains) can be administered, for example, 2-6 hours apart from the antibiotic.

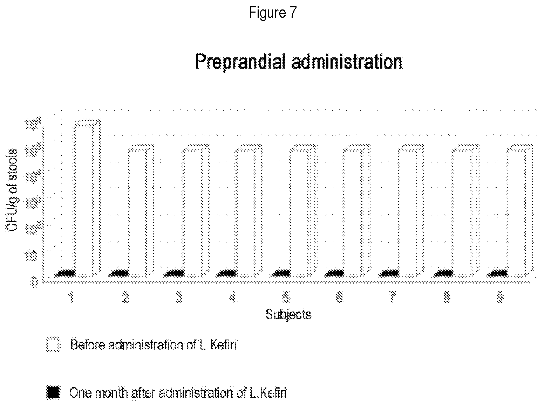

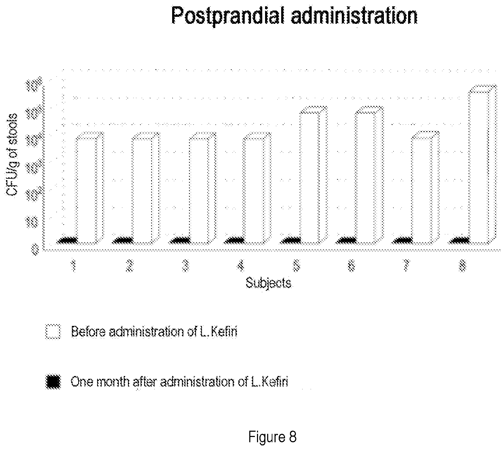

Advantageously, the bacterial strain *Lactobacillus kefiri* LKF01 DSM 32079 and/or bacterial strain *Lactobacillus kefiri* LKF02 DSM 32080 (or a composition for use of the present invention which contains said strains) can be administered on an empty or full stomach, either before or after meals; see Figures 7 and 8.

Another embodiment of the present invention envisages that the bacterial strain *Lactobacillus kefiri* LKF01 DSM 32079 and/or bacterial strain Lactobacillus kefiri LKF02 DSM 32080, for example from 200 to 500 mg each, in lyophilised form or in granules or in powder (preferably in a ratio by weight comprised from 1:2 to 2:1; 1:0, 0:1, 1:1), at a concentration comprised from 1x10⁸ CFU/g to 1x10¹² CFU/g of bacteria (preferably at a concentration comprised from 1x10⁹ CFU/g to 1x10¹¹ CFU/g of bacteria) are mixed, for example, with a maltodextrin, so as to form a composition/mixture. In this embodiment said bacterial strains are not coated, but are rather in the form of bare cells. This composition/mixture of said uncoated strains of *L. kefiri* is introduced into a rigid vegetable capsule selected from the ones known in the market. The capsule containing said composition/mixture is then in turn inserted and sealed in a sachet made from a material that is resistant to the passage of water, humidity, air, oxygen and light. The protection offered by the rigid vegetable capsule combined with the additional protection of the sachet together assure a perfectly isolated environment and a longer shelf life for the cells of the bacterial strain *Lactobacillus kefiri* LKF01 DSM 32079 and/or bacterial strain *Lactobacillus kefiri* LKF02 DSM 32080 contained inside said capsule. At the time of use, the content of said rigid vegetable capsule is added to a vegetable oil, such as, for example, sunflower oil, contained in a bottle, having a volume, for example, of 6 ml or 8 ml or 10 ml, to yield an oily suspension. The oily suspension can be administered in drops, for example by means of a dosing pipette. A dose, for example 5 drops, can be taken directly or mixed with foods or beverages that are cold, at room temperature (20 °C) or warm (30 °C), provided that they do not exceed 37 °C.

Embodiments FRn of the present description are disclosed here below.

A first embodiment FR1 relates to a food composition, or a composition for a supplement, or a composition for a medical device, or a pharmaceutical composition comprising excipients and/or substances of a food or pharmaceutical grade formulation, and a mixture which comprises or, alternatively, consists of a bacterial strain selected from the group consisting of the bacterial strains belonging to the species Lactobacillus kefiri, said composition being for use (i) in paediatrics to generate and/or maintain the state of homeostasis, and (ii) in adult subjects in the preventive and/or curative treatment of inflammatory intestinal diseases, such as irritable bowel syndrome (IBS), inflammatory bowel disease (IBD), Crohn's disease and ulcerative colitis (UC).

Another embodiment FR2 relates to a composition for use according to FR1, wherein said bacterial strain belonging to the species *Lactobacillus kefiri* is selected between the bacterial strain *Lactobacillus kefiri* LKF01 DSM 32079 deposited by Probiotical SpA on 10.07.2015 with the institute DSMZ - Germany, and the bacterial strain *Lactobacillus kefiri* LKF02 DSM 32080 deposited by Probiotical SpA on 10.07.2015 with the institute DSMZ - Germany; preferably, said strains are present in said composition in a ratio by weight comprised from 1 :2 to 2:1; 1:0, 0: 1, 1:1

Another embodiment FR3 relates to a composition for use according to either FR1 or FR2, wherein the bacterial cells of said bacterial strains are in a coated or microencapsulated form, with a coating material comprising or, alternatively, consisting, of at least one lipid of animal or vegetable origin. Another embodiment FR4 relates to a composition for use according to any one of FR1 , FR2 or FR3, wherein said coating material comprises or, alternatively, consists of at least one lipid of vegetable origin selected from the group comprising or, alternatively, consisting of saturated vegetable fats having a melting point comprised from 35 °C to 85 °C; preferably comprised from 45 °C to 70 °C, even more preferably from 50 °C to 60 °C.

Another embodiment FR5 relates to a composition for use according to any one of FR1, FR2, FR3 or FR4, wherein said lipid is selected from the group comprising or, alternatively, consisting of:
(i) Glyceryl dipalmitostearate E471, I NCI (PCPC): glyceryl distearate, CAS: 85251-77-0 (or 1323-83-7), EINECS: 286-490-9 (or 215-359-0); preferably Biogapress Vegetal BM 297 ATO -Gattefosse SAS;
(ii) Polyglyceryl palmitostearate E475, INCI: polyglyceryl-6-distearate, CAS: 61725-93-7; preferably Plural Stearique WL 1009 -Gattefosse SAS.

Another embodiment FR6 relates to a composition for use according to any one of FR1, FR2, FR3, FR4 or FR5, wherein said composition comprises or, alternatively, consists of a vegetable oil selected from the group comprising or, alternatively, consisting of an olive oil, a soybean oil, a corn oil, a rice oil or a sunflower oil, and said mixture of strains which comprises or, alternatively, consists of at least one bacterial strain selected from the group consisting of the bacterial strains belonging to the species *Lactobacillus kefiri,* as claimed in FR1-FR5.

Another embodiment FR7 relates to a composition for use according to any of the previous ones, FR1-FR6, wherein said bacterial strains, preferably in lyophilised form or in granules or in powder, are present in said composition at a concentration comprised from 1x10⁸ CFU/g to 1x10¹² CFU/g of composition, preferably at a concentration comprised from 1x10⁹ CFU/g to 1x10¹¹ CFU/g of composition.

Another embodiment FR8 relates to a composition for use according to FR6 or FR7, wherein the bacterial strains belonging to the species *L. kefiri,* in the form of a powder or a granulate or a lyophilisate to be added to a vegetable oil to yield a pharmaceutical form of an oily suspension or an oil in drops, are coated with a lipid such as polyglyceryl palmitostearate E475, INCI: polyglyceryl-6-distearate, CAS: 61725-93-7. Another embodiment FR9 relates to a composition for use according to FR8, wherein the bacterial strains are Lactobacillus kefiri LKF01 DSM 32079 and the bacterial strain Lactobacillus kefiri LKF02 DSM 32080, in a ratio by weight comprised from 1:2 to 2:1; 1:0, 0:1, 1:1.

Another embodiment FR10 relates to the bacterial strains *Lactobacillus kefiri* LKF01 DSM 32079 and Lactobacillus kefiri LKF02 DSM 32080, for use in paediatrics to generate and/or maintair the state of homeostasis and for use in the preventive and/or curative treatment of inflammatory intestinal diseases, such as irritable bowel syndrome (IBS), inflammatory bowel disease (IBD), Crohn's disease and ulcerative colitis (UC), in adult subjects, also in a chronic stage.

The Applicant conducted a comparative study to highlight the potentialities of the strains Lactobacillus kefiri LKF01 DSM 32079 LKEF and Lactobacillus kefiri LKF02 DSM 32080 in protecting and repairing the intestinal barrier. The comparative study was carried out versus other probiotic bacterial strains, as described below.

### Materials and methods

All of the strains used in this study were cultured at 37 °C in an elective medium - MRS - and then counted according to standard procedures with a cytofluorometry technique (FAC SCalibur flow cytometer manufactured by BD). Each strain was counted in a 10-³ dilution in saline solution using the Cell Viability Kit manufactured by the company BD, according to the standard procedure.

In this study, the reparative and/or protective activity performed by the bacterial strain Lactobacillus kefiri LKF01 DSM 32079 LKEF and the bacterial strain *Lactobacillus kefiri* LKF02 DSM 32080 was compared to that of the following bacterial strains:
(i) *Streptococcus thermophilus* ST10 DSM 25246,
(ii) *Streptococcus thermophilus* FP4 DSM 18616,
(iii) *Lactobacillus acidophilus* LA02 DSM 21717,
(iv) *Lactobacillus salivarius* LS01 DSM 22775,
(v) *Lactobacillus salivarius* LS03 DSM 22776,
(vi) *Lactobacillus salivarius* LS06 DSM 26037,
(vii) *Lactobacillus rhamnosus* GG ATCC 53103,
(viii) *Lactobacillus reuteri* LRE09 DSM 25685,
(ix) Mixture (1:1: 1:1:1) of *Bifidobacterium longum* DLBL 07 (DSM 25669), *Bifidobacterium longum* DLBL 08 (DSM 25670), *Bifidobacterium longum* DLBL 09 (DSM 25671), *Bifidobacterium longum* DLBL 10 (DSM 25672) and *Bifidobacterium longum* DLBL 11 (DSM 25673),
(x) Lactobacillus rahmnosus LR06 DSM 21981,
(xi) Lactobacillus delbrueckii ssp. delbrueckii 1DD01 ID1391 DSM 22106,
(xii) Lactobacillus plantarum LP01 LMG P-21021,
(xiii) *Lactobacillus fermentum* LF16 DSM 26956,
(xiv) *Bifidobacterium breve* BR03 DSM 16604,
(xv) *Bifidobacterium lactis* BS01 LMG P-21384 and
(xvi) *Bifidobacterium longum* W11 LMG P-21586.

All of the above strains (i) to (xvi) are defined as the bacterial strains of the present study.

Use was made of an in vitro model simulating the intestinal barrier, to which damage was induced using pro-inflammatory cytokines, namely, TNF-alpha and IL-1 beta.

The experimentation provided for the use of 24-multiwell transwell plates, in which CACO-2 cells were cultured for 21 days until forming a confluent layer of cells, using an in vitro model of intestinal epithelial tissue (manufacturer ARETA International, Gerenzano -Varese (VA) Italy). All of the experiments were conducted in a DMEM medium with 10% FBS (Fetal Bovine Serum -GIBCO). Two types of experimental protocols were carried out in parallel.

### Protocol for assessing protection/restoration by the strains studied

The ability to control the invasion of external agents via the intestinal lumen is called the "barrier function of the intestinal mucosa" (2-3). The integrity of this barrier function can be measured by trans-epithelial electrical resistance (Ω/cm²), or TEER. TEER is an in vitro measurement of the passage of ions through the paracellular pathway. Maintaining intestinal integrity is critical for the basal physiological processes of the intestine. Therefore, a reduction in TEER values can represent an early expression of cell damage and indicate that the barrier is compromised (4-5). In order to assess the efficiency of the strains on the intestinal barrier, two assessment protocols were used: a first protocol for assessing protection and a second protocol relating to membrane restoration (6).

The first protocol related to the assessment of protection entails, after a reading of the initial resistance in each well, a pre-incubation of the CACO-2 cells with the probiotic bacterial strains of the present study (concentration 3x10⁶ cells/well) at 37 °C for 1 hour, followed by one hour of incubation with pro- inflammatory stimuli: TNF-alpha and IL-1beta (produced by Immunotools) used at the final concentration of 10 ng/ml), stimuli which simulate damage to the intestinal epithelial barrier.

In this first experimental protocol, the aim was to assess the ability of the probiotic bacterial strains of the present study (i)-(xvi) in protecting the membrane's integrity upon the arrival of a pro-inflammatory insult (Protection Protocol)

The second protocol related to the assessment of the restoration of barrier functionality entails, after a reading of the initial resistance in each well, 1 hour of pre-incubation of the CACO-2 cells with pro-inflammatory stimuli which induce epithelial damage: TNF-alpha and IL-1 beta (produced by Immunotools) at the final concentration of 10ng/ml, followed by incubation with the probiotics (3x10⁶ cells/well).

In this second experimental protocol, the aim was to assess, in the in vitro model, the ability of the probiotic bacterial strains of the present study to restore membrane integrity after damage induced by a pro-inflammatory stimulus (Restoration Protocol).

### Assessment of TEER

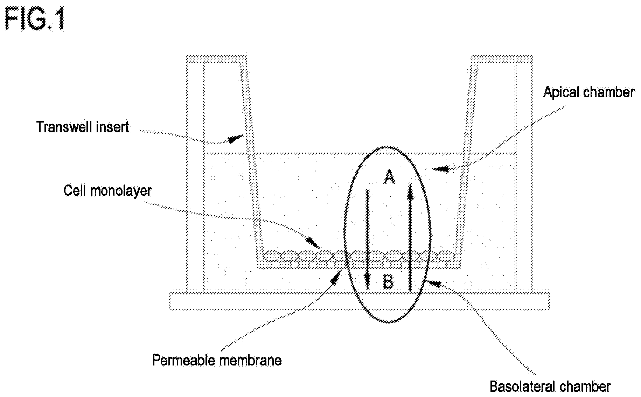

In both protocols (6), 2 hours after stimulation the medium in the apical chamber and basolateral chamber was changed. TEER (Trans-Epithelial Electrical Resistance) was evaluated in the experimental cellular model 24 hours after stimulation using an EVOM2, an instrument specifically designed to measure trans-epithelial electrical resistance in cell tissues and capable of performing a correct measurement of the electrical resistance between the *apical chamber* and the *basolateral chamber* -see Figure 1. The TEER measurements are presented as a percentage of the TEER values at the start of the experimentation. Furthermore, as a first step, in order to verify the interaction between the bacterial strains of the present study (i)-(xvi) and the epithelium, the transwell supernatants collected at the end of the 2 hours of stimulation were used with the aim of assessing the adhesion of the bacterial strains of the present study to the epithelial layer. The assessment of adhesion was conducted indirectly via cytofluorometric analysis of the count of the bacterial strains remaining in the collected supernatant.

### Protocol for assessing the adhesion of the strains studied to CACO-2 cells

In order to verify the interaction between the bacterial strains and underlying epithelium formed by the CACO-2 cells, a flow cytometer was used to count the bacteria present in the supernatants of the apical chambers collected at the end of the 2 hours of stimulation, with a modification of a protocol to assess adhesion on plates already present in the literature (7). The value obtained is the number of cells which did not adhere to the epithelium. The adhesion value of is an indirect value, calculated as a percentage relative to the number of the bacterial cells used at the start of the experimentation.

Results: Assessment of adhesion to intestinal cells (comparative assessment of the adhesion of *L.Kefiri* LKF01 (DSM 32079) and other bacterial strains to intestinal epithelial cells (CACO-2)).

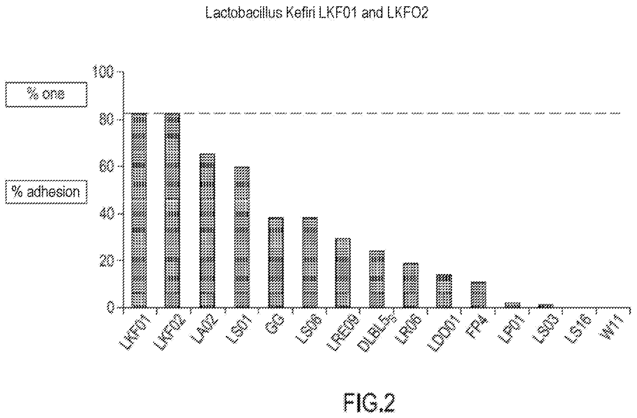

All of the bacterial strains considered in the present study were left to incubate for 2 hours at 37 °C with CACO-2 cells at confluence in order to study the interaction of the bacteria with the underlying cells. As can be seen from Figure 2, which shows the percentage of adhesion of some bacterial strains of the present study vis-a-vis CACO-2 cells, the totality of the bacterial strains of the present study tested in comparison with the bacterial strain *Lactobacillus kefiri* LKF01 DSM 32079 showed to be less effective in adhering to the epithelial layer formed by the CACO-2 cells.

As can be inferred from Figure 2 (adhesion to intestinal cells), the bacterial strain *Lactobacillus kefiri* LKF01 DSM 32079 LKEF surprisingly shows a very strong, marked adhesion to the underlying epithelium compared to the tested bacterial strains. It should be noted that the bacterial strains of the present study which did not show any detectable adhesion are not included in Figure 2.

This very pronounced adhesion of the bacterial strain *Lactobacillus kefiri* LKF01 DSM 32079 prompted the Applicant to carry out the second part of the experimentation as well, which entailed measuring the trans-epithelial electrical resistance (TEER) in every well included in the Protection Protocol (pre-stimulation with the strains of interest + stimulation with pro-inflammatory factors) and Restoration Protocol (pre-stimulation with pro-inflammatory factors + subsequent stimulation with the bacterial strains).

### Protocol related to the protection of intestinal cells against inflammatory damage (probiotic strains + pro-inflammatory stimuli)

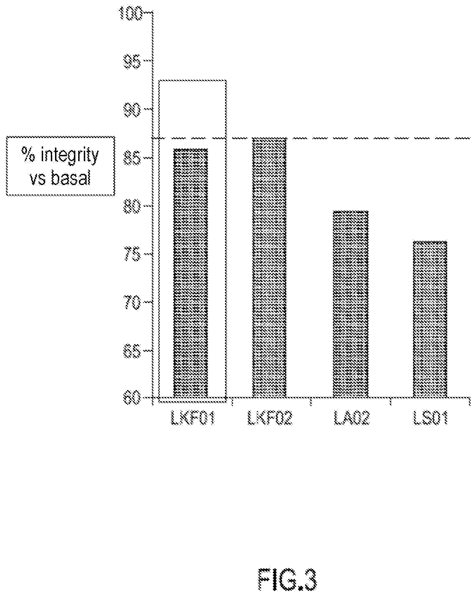

In the Protection Protocol, all of the tested strains demonstrated an ability to maintain membrane integrity above 70% versus the control (basal TEER value of the epithelial cells). Furthermore, the protective action of the bacterial strain *Lactobacillus kefiri* LKF01 DSM 32079 LKEF and the bacterial strain *Lactobacillus kefiri* LKF02 DSM 32080 demonstrated to be comparable and/or better than that of the following bacterial strains:
(i) *Streptococcus thermophilus* ST10 DSM 25246,
(iii) *Lactobacillus acidophilus* LA02 DSM 217170,
(iv) *Lactobacillus salivarius* LS01 DSM 22775 (*),
(v) *Lactobacillus salivarius* LS03 DSM 22776,
(viii) *Lactobacillus reuteri* LRE09 DSM 25685,
(ix) Mixture (1 :1 : 1 :1 :1) of *Bifidobacterium longum* DLBL 07 (DSM 25669), *Bifidobacterium longum* DLBL 08 (DSM 25670), *Bifidobacterium longum* DLBL 09 (DSM 25671), *Bifidobacterium longum* DLBL 10 (DSM 25672) and *Bifidobacterium longum* DLBL 11 (DSM 25673),
(xi) *Lactobacillus delbrueckii ssp. delbrueckii* 1DD01 ID1391 DSM 22106,
(xvi) *Bifidobacterium longum* W11 LMG P-21586.
(*) Data shown in Figure 3.

Figure 3 (Protection of intestinal cells against inflammatory damage) relates to the assessment of the degree of protection of intestinal cells (CACO-2) pre-treated with the bacterial strain *Lactobacillus kefiri* LKF01 DSM 32079 LKEF and the bacterial strain *Lactobacillus kefiri* LKF02 DSM 32080 and other bacterial strains before a pro-inflammatory stimulus (cytokines TNF-a and I L-1 b).

As can be inferred, therefore, from Figure 3 [Percentage of membrane integrity assessed versus the basal TEER value of non-stimulated CACO-2 (basal value equal to 100%; pro-inflammatory stimulus equal to 40%; reading at 24 hours)] the pre-treatment with the bacterial strain *Lactobacillus kefiri* LKF01 DSM 32079 and the bacterial strain *Lactobacillus kefiri* LKF02 DSM 32080, prior to a pro-inflammatory stimulus capable of reducing the TEER values to 40% compared to the basal value, helps to preserve the integrity of the epithelial layer formed by the CACO-2 cells, as it maintains the percentage of integrity at 86%.

Figure 3 shows the percentage of integrity maintained by the epithelium after pre-treatment with the three strains which show greater adhesion. The pre-treatment with the bacterial strain *Lactobacillus kefiri* LKF01 DSM 32079 and the bacterial strain *Lactobacillus kefiri* LKF02 DSM 32080, prior to a pro-inflammatory stimulus capable of reducing the integrity of the intestinal barrier by up to 40% compared to the basal value, helps to preserve the integrity of the epithelial layer formed by the CACO-2 cells, as it maintains the percentage of membrane integrity at 86%.

### Restoration Protocol (pro-inflammatory stimuli + probiotic strains)

We then proceeded to test whether the strains considered were capable of restoring the integrity of the membrane damaged by pre-incubation with the pro-inflammatory cytokines IL-1 beta and TNF-alpha.

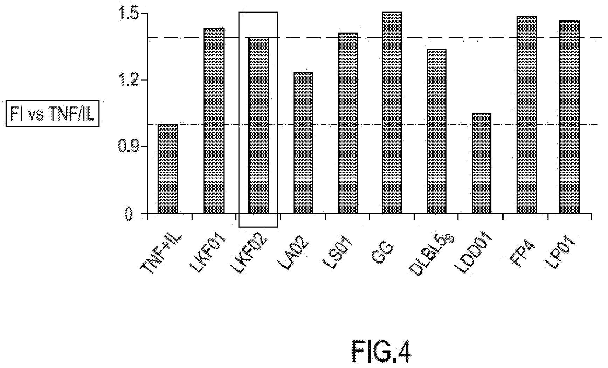

As can be inferred from figure 4 [fold increase (FI) in membrane integrity measured against the TEER value of CACO-2 cells stimulated with pro-inflammatory factors (TEER value of CACO-2 cells stimulated with pro-inflammatory stimuli equal to 1; reading at 24 hours)].

24 hours after the inflammatory insult, all of the tested strains contributed to restoring the integrity of the membrane damaged by the inflammatory stimulus and as can be seen from figure 4, the bacterial strain *Lactobacillus kefiri* LKF01 DSM 32079 LKEF and the bacterial strain *Lactobacillus kefiri* LKF02 DSM 32080, as well as other strains, were capable of bringing the TEER values back to the basal ones (non-stimulated CACO-2).

These data demonstrate that using the strains in question to treat the epithelium formed by the CACO-2 cells and damaged by the presence of pro-inflammatory stimuli restored the basal TEER values and that several strains, including the bacterial strain *Lactobacillus kefiri* LKF01 DSM 32079 and the bacterial strain *Lactobacillus kefiri* LKF02 DSM 32080, lead to an improvement in membrane integrity.

From the foregoing, it emerges that the bacterial strain

*Lactobacillus kefiri* LKF01 DSM 32079 and the bacterial strain *Lactobacillus kefiri* LKF02 DSM 32080, isolated from kefir, show an extraordinary and surprising ability to adhere to the epithelium.

Furthermore, the bacterial strain *Lactobacillus kefiri* LKF01 DSM 32079 and the bacterial strain *Lactobacillus kefiri* LKF02 DSM 32080 show a dual action: (i) a protective action and (ii) an action of restoring the intestinal epithelium after pro-inflammatory stimulation due to the presence of the cytokines TNF-alpha and IL-1 beta.

The above-demonstrated properties support the use of the bacterial strains Lactobacillus kefiri LKF01 DSM 32079 and/or Lactobacillus kefiri LKF02 DSM 32080 in paediatrics to generate and/or maintain the state of homeostasis and the use in the preventive and/or curative treatment of inflammatory intestinal diseases, such as irritable bowel syndrome (IBS), inflammatory bowel disease (IBD), Crohn's disease and ulcerative colitis (UC), in adult subjects, also in a chronic stage.

The Applicant also conducted an assay for the recovery of the bacterial strain *Lactobacillus kefiri* LKF01 DSM 32079 LKEF from human stool samples.

### DNA extraction

Total DNA was extracted from stool samples one month after administration using the QIAamp DNA Stool Mini Kit in accordance with the instructions of the manufacturer (Qiagen, Italy). The protocol included a specific binding of the DNA to the QIAmp silica-gel membrane while the contaminants pass through.

### Quantitative Real-Time PCR

In order to quantify the bacterial strain *Lactobacillus kefiri* LKF01 DSM 32079 in stool samples, the following primers were used:
- LK1 5' CAA CAA TCA AAG GGG TTG TTG 3';
- LK2 5' TCA CTA GGA GTA ATT GAA CCA 3'.

Real-time PCR was performed using the Rotor-Gene RG 3000A detection system with a fluorescence detector of the SYBR green dye type. The amplification consisted in maintenance at 95 °C for 10 minutes followed by 35 cycles at 95 °C for 60 seconds, 50 °C for 60 seconds, 72 °C for 60 seconds and final maintenance at 72 °C for 10 minutes. For the standards, serial (10-fold) dilutions of the pure cultures of the target bacteria were followed by DNA extraction for each dilution, so as to obtain a linear relationship between the Ct and number of bacteria.

The Applicant carried out a study on the colonisation of the intestine with specific reference to a clinical assessment performed on 20 subjects (4 males and 16 females, mean age 40 years) divided into two groups: 10 subjects with preprandial administration and 10 subjects with postprandial administration for one month, 5 drops per day of a vegetable oil-based composition.

Figure 7 refers to the administration of the 5-drop dose of said oil-based composition 15-30 minutes before breakfast in 10 subjects for one month. Only one subject left the study because of taking antibiotic therapy at the 21st (twenty-first) day. Whereas Figure 8 refers to the administration of the 5-drop dose of said oil-based composition after the meal in 10 subjects for one month. Two subjects left the study because of inconsistent intake of the dose.

One month after administration of the strain *L kefiri* LKF01 (DSM 32079), the microorganism was found in the stools of all subjects involved in the study, after both preprandial and postprandial administration.

These data reveal the high colonisation capacity of the strain *L.kefiri* LKF01 (DSM 32079), which is capable not only of surviving in the gastric environment, but also of persisting and continuing to be viable in the human intestine.

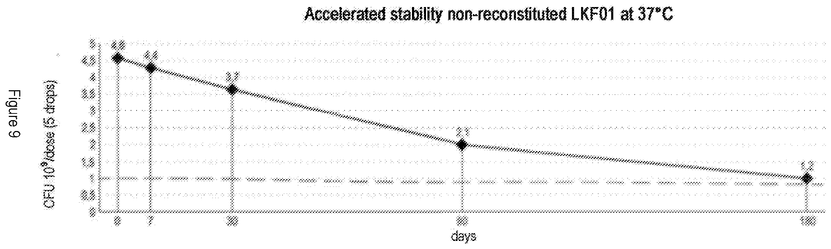

Several stability tests were also performed, Figures 9-10-11.

A first stability test (Figure 9) refers to the assessment of the stability of the strain Lkefiri LKF01 (DSM 32079) in the intact capsule package (not dispersed in oil) subjected to a constant temperature of 37 °C for up to 6 months, Figure 9. The data shown in Figure 9 show that a week of continual exposure to 37 °C does not significantly affect the stability of the composition/strain. One month of exposure to 37 °C is practically identical to one month of the same microorganism dispersed in oil at 25 °C.

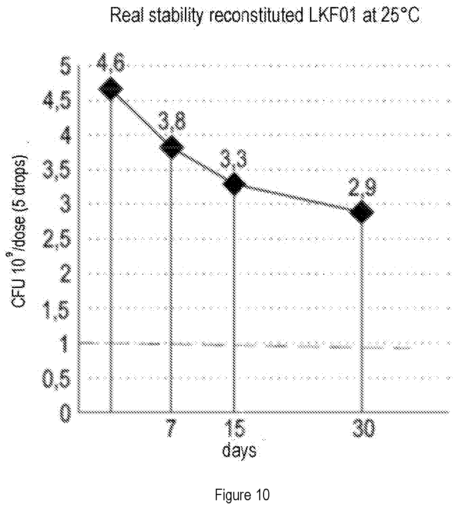

A second stability test (Figure 10) refers to the assessment of *L.kefiri* LKF01 (DSM 32079) over time, from the moment of dispersion of the contents of the capsule in the oil contained in the bottle up to one month later, Figure 10. The data shown in Figure 10, with reference to 30 days of contact at 25 °C, reveal an excellent stability of the non-microencapsulated microorganism (bare cells) dispersed in oil. In particular, they confirm the nearly total membrane integrity (cytofluorometry assessment), meaning that the microorganism is not structurally affected by the presence in oil. Therefore, the probiotic cell content/concentration of the strain *L.kefiri* LKF01 (DSM 32079), as declared on the final product to be administered, is guaranteed after a month of its presence in vegetable oil.

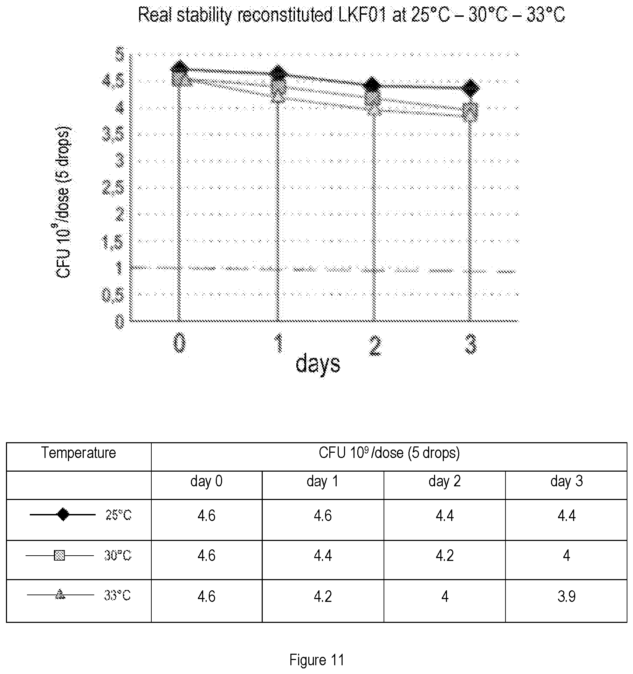

A third stability test (Figure 11) refers to the assessment of *L.kefiri* LKF01 (DSM 32079) from the moment of dispersion of the contents of the capsule in the oil contained in the bottle, incubated at 3 different temperatures (25 °C, 30 °C, 33 °C) for 3 days, Figure 11. The data shown in Figure 11 demonstrate an excellent survival of the microorganism even when subjected to storage temperatures above the recommended ones for 3 days.

### Strain characterization

| Strain | *Lactobacillus kefiri* |
|---|---|
| Internal identification number - ID | ID 1927 |
| Probiotical commercial code: | LKF01 |
| Deposition number in DMZ international collection | DSM 32079 |

### Strain characterization

### Origin

The strain *Lactobacillus kefiri* was isolated from Kefir granules.

All analyses were conducted in the laboratories of Biolab Research srl, a subsidiary of the Mofin Alce Group, affiliate of Probiotical S.p.A

### Biochemical characterisation

1) Sugar fermentation profile (API 50 CHL, Bio Merieux)

| **N.** | **Sugars** | **Results** | **N.** | **Sugars** | **Results** |
|---|---|---|---|---|---|
| **0** | Control | - | **25** | Esculin ferric citrate | - |
| **1** | Glycerol | - | **26** | Salicin | - |
| **2** | *Erythritol* | - | **27** | D-Cellobiose | - |
| **3** | D-Arabinose | - | **28** | D-Maltose | **+** |
| **4** | L-Arabinose | **+** | **29** | D-Lactose | - |
| **5** | D-Ribose | **+** | **30** | D-Melibiose | - |
| **6** | D-Xylose | **-** | **31** | D-Sucrose | - |
| **7** | L-Xylose | **-** | **32** | D-Trehalose | - |
| **8** | Adonitol | **-** | **33** | Inulin | - |
| **9** | Methyl-βD-Xylopyranoside | **-** | **34** | D-Melezitose | - |
| **10** | D-Galactose | **-** | **35** | D-Raffinose | - |
| **11** | D-Glucose | **+** | **36** | Starch | - |
| **12** | D-Fructose | **-** | **37** | Glycogen | - |
| **13** | D-Mannose | **-** | **38** | Xylitol | - |
| **14** | L-Sorbose | - | **39** | Gentiobiose | - |
| **15** | L-Rhamnose | - | **40** | D-Turanose | - |
| **16** | Dulcitol | - | **41** | D-Lyxose | - |
| **17** | Inositol | - | **42** | D-Tagatose | |
| **18** | D-Mannitol | - | **43** | D-Fucose | - |
| **19** | D-Sorbitol | - | **44** | L-Fucose | - |
| **20** | Methyl-αD-Mannopyranoside | - | **45** | D-Arabitol | - |
| **21** | Methyl-αD-Glucopyranoside | - | **46** | L-Arabitol | - |
| **22** | N-Acetyl-Glucosamine | - | **47** | Potassium gluconate | **+** |
| **23** | Amygdalin | - | **48** | Potassium 2-Ketogluconate | - |
| **24** | Arbutin | - | **49** | Potassium 5-Ketogluconate | - |

2) Enzymatic profile (API Zym Bio Mérieux)

| **N.** | **Sublayer** | **Enzymes analysed** | **Type of enzyme** | **Activity** (napomoles) |
|---|---|---|---|---|
| **1** | Negative control | - | - | - |
| **2** | 2-naphthyl phosphate | Alkaline phosphatase | **PHOSPHATASE** | **5** |
| **3** | 2-naphthyl butyrate | Esterase (C 4) | LIPASE | - |
| **4** | 2-naphthyl caprylate | Esterase lipase (C 8) | | **10** |
| **5** | 2-naphthyl myristate | Lipase (C 14) | | - |
| **6** | L-leucyl-2-naphthylamide | *Leucine arylamidase* | PROTEASE | ≥**40** |
| **7** | L-valyl-2-naphthylamide | Valine *arylamidase* | | **20** |
| **8** | L-cystyl-2-naphthylamide | Cystine arylamidase | | **10** |
| **9** | N-benzoyl-DL-arginine-2-naphthylamide | Trypsin | | - |
| **10** | N-glutaryl-phenylalanine-2-naphthylamide | α-chymotrypsin | | - |
| **11** | 2-naphthyl phosphate | Acid phosphatase | PHOSPHATASE | **20** |
| **12** | Naphthol-AS-BI-phosphate | Naphthol-AS-BI-phosphohydrolase | | **5** |
| **13** | 6-Br-2-naphthyl-αD-galactopyranoside | α*-galactosidase* | OXIDASE | **30** |
| **14** | 2-naphthyl-βD-galactopyranoside | β*-galactosidase* | | **≥40** |
| **15** | Naphthol-AS-BI-βD-glucuronide | β-glucuronidase | | **20** |
| **16** | 2-naphthyl-αD-glucopyranoside | α-glucosidase | | **30** |
| **17** | 6-Br-2-naphthyl-βD-glucopyranoside | β-glucosidase | | - |
| **18** | 1-naphthyl-N-acetyl-βD-*glucosaminide* | N-acetyl-β-glucosaminidase | | - |
| **19** | 6-Br-2-naphthyl-αD-mannopyranoside | α-mannosidase | | - |
| **20** | 2-naphthyl-αL-fucopyranoside | α-fucosidase | | - |

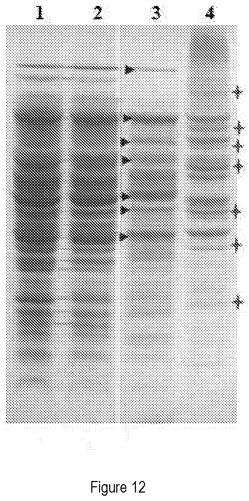

3) Protein profile (Electrophoresis on polyacrylamide gel - Figure 12) where :
1. Strain: *L. kefiri* _ LKF01 (ID 1927) - Master Cell Bank (MCB origin)
2. Strain: *L. kefiri _* LKF01 (ID 1927) - Master Cell Bank (6^{th} sub-culture)
3. Positive reference: *L. kefiri -* DSM 20587
4. Negative reference : *B.infantis* BI05 - DSM 28652

Pathways 1-2: Protein profiles of the Master Cell Bank cultures of the strain; (see lines);
Pathways 1-2 and 3: The profile of the strain is characteristic of the species Lactobacillus kefiri (see arrows);
Pathways 4 and 1-2: The protein profile of the strain is different from the one obtained from a strain belonging to a different species (*Bifidobacterium infantis* DSM 28652 ; see crosses).

### Molecular characterisation

### Species-specific classification:

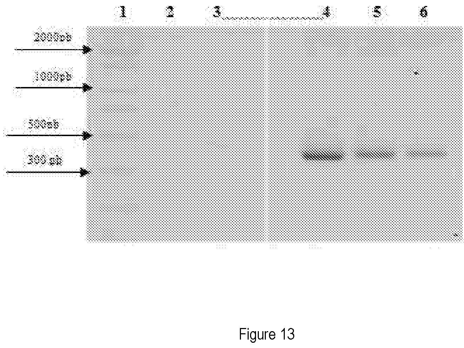

### 4) Polymerase chain reaction (PCR) using the primers LK1/ LK2 (Figure 13)

### Positive reaction for Lactobacillus kefiri where:

1. PCR Marker: Sigma 50-2000 bp
2. Blank: No DNA
3. Negative reference: *L buchneri* DSM 20057
4. Positive reference: *L kefiri* DSM 20587
5. Strain: *L kefiri* _ LKF01 ID 1927
6. Strain: *L kefiri_*LKF01 ID 1927

### 5) 16S rDNA gene sequencing

Results obtained with the Blast program (http://blast.ncbi.nlm.nih.gov/Blast.cgi)

| | | | | |
|---|---|---|---|---|
| Length=1501 | | | | |

| Sequences producing significant alignments | | | Score (Bits) | E Value |
|---|---|---|---|---|
| dbj | \|AB429371.1\| | Lactobacillus kefiri gene for 16S rRNA, parti... | 2690 | 0.0 |
| gb | \|KJ128236.1\| | Lactobacillus kefiri strain DA-84 16S ribosoma... | 2682 | 0.0 |
| gb | \|AY363303.1\| | Lactobacillus kefiri 16S ribosomal RNA gene, p... | 2679 | 0.0 |
| gb | \|KJ128235.1\| | Lactobacillus kefiri strain DA-46 16S ribosoma... | 2673 | 0.0 |
| dbj | \|AB362680.1\| | Lactobacillus kefiri gene for 16S rRNA, parti... | 2672 | 0.0 |
| gb | \|KC755081.1\| | Uncultured Lactobacillus sp. clone 6c19 16S ri... | 2657 | 0.0 |
| dbj | \|AB362681.1\| | Lactobacillus kefiri gene for 16S rRNA, parti... | 2655 | 0.0 |
| gb | \|HM218239.1\| | Lactobacillus kefiri strain NM54-2 16S ribosom... | 2650 | 0.0 |
| gb | \|KF263157.1\| | Lactobacillus kefiri strain KG-17 16S ribosoma... | 2639 | 0.0 |
| gb | \|KF234766.1\| | Lactobacillus kefiri strain K-22 16S ribosomal... | 2639 | 0.0 |

Score: Number used to evaluate the biological relevance of an identification. In sequence alignments, the score is a numerical value which describes the overall quality of an alignment. Higher numbers correspond to greater similarity.

E-value (expected value): a value which, if correctly interpreted by a researcher, will indicate the likelihood of a score indicating a correlation between the two biological sequences. The lower the E-value, the more significant the score.

### Genetic profile

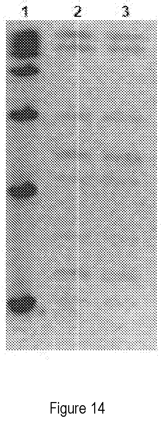

### 6) Pulsed-field gel electrophoresis (PFGE- Figure 14) with Not I where:

1. Marker: Sigma 50-1,000 kb
*2. L. kefiri* LKF 01 ID 1927- origin -Master Cell Bank
*3. L. kefiri* LKF01 ID 1927-6th sub-culture - Master Cell Bank

### Strain characterization

| Stain: | *Lactobacillius kefiri* |
|---|---|
| Internal identification number -ID: | ID 1911 |
| Probiotical commercial code: | LKF02 |
| Deposition number in DSMZ international collection | DSM 32080 |

### Origin

### The strain Lactobacillus kefiri was isolated from Kefir granules

All analyses were conducted in the laboratories of Biolab Research srl, a subsidiary of the Mofin Alee Group and affiliate of Probiotical S.p.A.

### Biochemical characterisation

1) Sugar fermentation profile (API 50 CHL, Bio Merieux)

| **N.** | **Sugar** | **Results** | **N.** | **Sugars** | **Results** |
|---|---|---|---|---|---|
| **0** | Control | - | **25** | Esculin ferric citrate | + |
| **1** | Glycerol | - | **26** | Salicin | - |
| **2** | *Erythritol* | - | **27** | D-Cellobiose | - |
| **3** | D-Arabinose | - | **28** | D-Maltose | + |
| **4** | L-Arabinose | + | **29** | D-Lactose | + |
| **5** | D-Ribose | + | **30** | D-Melibiose | + |
| **6** | D-Xylose | - | **31** | D-Sucrose | - |
| **7** | L- Xylose | - | **32** | D-Trehalose | - |
| **8** | Adonitol | - | **33** | Inulin | - |
| **9** | Methyl-βD-Xylopyranoside | - | **34** | D-Melezitose | - |
| **10** | D-Galactose | + | **35** | D-Raffinose | + |
| **11** | D-Glucose | + | **36** | Starch | - |
| **12** | D-Fructose | + | **37** | Glycogen | - |
| **13** | D-Mannose | - | **38** | Xylitol | - |
| **14** | L-Sorbose | - | **39** | Gentiobiose | - |
| **15** | L-Rhamnose | - | **40** | D-Turanose | - |
| **16** | Dulcitol | - | **41** | D-Lyxose | - |
| **17** | Inositol | - | **42** | D-Tagatose | - |
| **18** | D-Mannitol | - | **43** | D-Fucose | - |
| **19** | D-Sorbitol | - | **44** | L-Fucose | - |
| **20** | Methyl-αD-Mannopyranoside | - | **45** | D-Arabitol | - |
| **21** | Methyl-αD-Glucopyranoside | - | **46** | L-Arabitol | - |
| **22** | N-Acetyl-Glucosamine | - | **47** | Potassium gluconate | + |
| **23** | Amygdalin | - | **48** | Potassium 2-Ketogluconate | - |
| **24** | Arbutin | - | **49** | Potassium 5-Ketogluconate | - |

2) Enzymatic profile (API Zym, Bio Merieux)

| **N.** | **Subiayer** | **Enzymes analysed** | **Type of enzyme** | **Activity** (nanomoles) |
|---|---|---|---|---|
| **1** | Negative control | - | - | - |
| **2** | 2-naphthyl phosphate | Alkaline phosphatase | **PHOSPHATASE** | - |
| **3** | 2-naphthyl butyrate | Esterase (C 4) | LIPASE | - |
| **4** | 2-naphthyl caprylate | Esterase lipase (C 8) | | **5** |
| **5** | 2-naphthyl myristate | Lipase (C 14) | | - |
| **6** | L-leucyl-2-naphthylamide | *Leucine arylamidase* | PROTEASE | **≥40** |
| **7** | L-valyl-2-naphthylamide | Valine *arylamidase* | | **20** |
| **8** | L-cystyl-2-naphthylamide | Cystine arylamidase | | **10** |
| **9** | N-benzoyl-DL-arginine-2-naphthylamide | Trypsin | | - |
| **10** | N-glutaryl-phenylalanine-2-naphthylamide | α-chymotrypsin | | - |
| **11** | 2-naphthyl-phosphate | Acid phosphatase | PHOSPHATASE | **≥40** |
| **12** | Naphthol-AS-BI-phosphate | Naphthol-AS-BI-phosphohydrolase | | **5** |
| **13** | 6-Br-2-naphthyl-αD-galactopyranoside | α*- galactosidase* | OXIDASE | **≥40** |
| **14** | 2-naphthyl-βD-galactopyranoside | β*-galactosidase* | | **≥40** |
| **15** | Naphthol-AS-BI-BD-glucuronide | β-glucuronidase | | **20** |
| **16** | 2-naphthyl-αD-glucopyranoside | α-glucosidase | | **30** |
| **17** | 6-Br-2-naphthyl-βD-glucopyranoside | β-glucosidase | | **-** |
| **18** | 1-naphthyl-N-acetyl-βD-*glucosaminide* | N-acetyl-β-glucosaminidase | | - |
| **19** | 6-Br-2-naphthyl-αD-mannopyranoside | α-mannosidase | | - |
| **20** | 2-naphthyl-αL-fucopyranoside | α-fucosidase | | - |

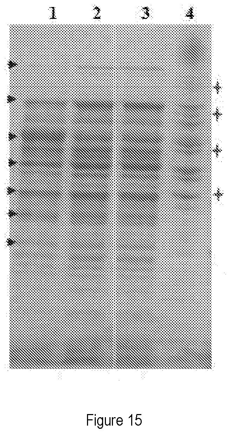

3) Protein profile (Electrophoresis on polyacrylamide gel - Figure 15) where:
1. Positive reference: *L. kefiri -* DSM 20587
2. Strain: *L. kefiri* _ LKF02 (ID 1911) - Master Cell Bank (origin)
3. Strain: L. kefiri _ LKF02 (ID 1911) - Master Cell Bank (sub-culture 13#6)
4. Negative reference *B.infantis* BI05 - DSM 28652
   Pathways 2-3: The protein profiles of the Master Cell Bank cultures of the strain; (see lines);
   Pathways 1 and 2-3: The profile of the strain is characteristic of the species *Lactobacillus kefiri* (see arrows);
   Pathways 4 and 2-3: The protein profile of the strain is different from the one obtained from a strain belonging to a different species (*Bifidobacterium infantis* DSM 28652; see crosses).

### Molecular characterisation

### Species-specific classification:

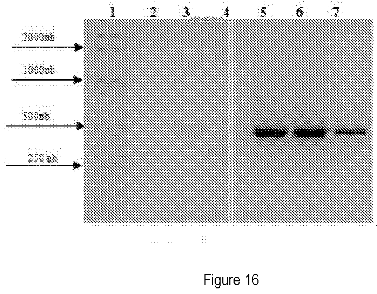

### 4) Polymerase Chain Reaction (PCR - Figure 16) using the primers LK1/ LK2

### Positive reaction for Lactobacillus kefiri

| | |
|---|---|
| 1. PCR Marker: | Sigma 50-3000 bp |
| 2. Blank: | No DNA |
| 3. Negative reference: | *L buchneri* DSM 20057 |
| 4. Negative reference: | *L fermentum* DSM 20052 |
| 5. Positive reference: | *L kefiri* DSM 20587 |
| 6. Strain: | *L. kefiri* _LKF02 ID 1911 |
| 7. Strain: | *L. kefiri* _LKF02 ID 1911 |

### 5) 16S rDNA gene sequencing

Results obtained with the Blast program
(http://blast.ncbi.nlm.nih.gov/Blast.cgi).

| Length=1541 | | | | |
|---|---|---|---|---|
| Sequences producing significant alignments | | | Score (Bits) | E Value |
| dbj | \|AB429371.1\| | Lactobacillus kefiri gene for 16S rRNA, parti... | 2773 | 0.0 |
| gb | \|AY363303.1\| | Lactobacillus kefiri 16S ribosomal RNA gene, p... | 2755 | 0.0 |
| dbj | \|AB362680.1\| | Lactobacillus kefiri gene for 16S rRNA, parti... | 2749 | 0.0 |
| dbj | \|AB362681.1\| | Lactobacillus kefiri gene for 16S rRNA, parti... | 2742 | 0.0 |
| gb | \|KJ128236.1\| | Lactobacillus kefiri strain DA-84 16S ribosoma ... | 2738 | 0.0 |
| gb | \|KJ128235.1\| | Lactobacillus kefiri strain DA-46 16S ribosoma ... | 2738 | 0.0 |
| gb | \|KC755081.1\| | Uncultured Lactobacillus sp. clone 6c19 16S ri... | 2726 | 0.0 |
| gb | \|HM218239.1\| | Lactobacillus kefiri strain NM54-2 16S ribosom... | 2726 | 0.0 |
| gb | \|KF263157.1\| | Lactobacillus kefiri strain KG-17 16S ribosoma ... | 2717 | 0.0 |
| gb | \|KF234766.1\| | Lactobacillus kefiri strain K-22 16S ribosomal ... | 2717 | 0.0 |

Score: Mumber used to evaluate the biological relevance of an identification. In sequence alignments, the score is a numerical value which describes the overall quality of an alignment. Higher numbers correspond to greater similarity.

E-value (expected value): a value which, if correctly interpreted by a researcher, will indicate the likelihood of a score indicating a correlation between the two biological sequences. The lower the E-value, the more significant the score.

### Genetic profile

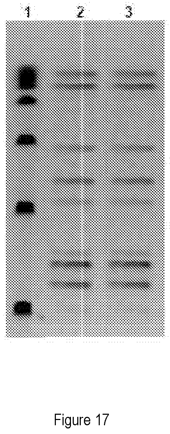

6 ) Pulsed-field gel electrophoresis (PFGE -Figure 17) with Not I
where:
1. Electrophoretic Marker: Sigma 50-1 ,000 kb
*2. L. kefiri* LKF02 ID 1911- culture origin -Master Cell Bank
*3. L. kefiri* LKF02 ID 1911-6th sub-culture - Master Cell Bank

### REFERENCES

1. Mogna L, Del Piano M, Deidda F, Nicola S, Soattini L, Debiaggi R, Sforza F, Strozzi G, Mogna G. Assessment of the in vitro inhibitory activity of specific probiotic bacteria against different Escherichia coli strains. J Clin Gastroenterol. 2012
2. Marchiando AM, Graham WV, Turner JR. Epithelial barriers in homeostasis and disease. Annu Rev Pathol. 2010;5: 119-44.
3. Farhadi A, Banan A, Fields J, Keshavarzian A. Intestinal barrier: an interface between health and disease. J Gastroenterol Hepatol. 2003 May; 18(5):479-97.
4. Ohland CL1 , Macnaughton WK. Probiotic bacteria and intestinal epithelial barrier function. Am J Physiol Gastrointest Liver Physiol. 2010 Jun;298(6):G807-19.
5. Miyauchi E, O'Callaghan J, Butto LF, Hurley G, Melgar S, Tanabe S, Shanahan F, Nally K, O'Toole PW. Mechanism of protection of transepithelial barrier function by Lactobacillus salivarius: strain dependence and attenuation by bacteriocin production. Am J Physiol Gastrointest Liver Physiol. 2012 Nov 1;303(9):G1029-41
6. Van Hemert S, Ormel G. Influence of the multispecies probiotic ecologic BARRIER on parameters of intestinal barrier function. Food and Nutrition Sciences 2014, 5, 1739-1745
7. Kebouchi M, et al. Implication of sortase-dependent proteins of Streptococcus thermophilus in adhesion to human intestinal epithelial cell lines and bile salt tolerance. Appl Microbiol Biotechnol. 2016 Jan 28.

## Claims

1. Bacterial strains belonging to the species Lactobacillus kefiri identified as *Lactobacillus kefiri* LKF01 DSM 32079, deposited by Probiotical SpA on 10.07.2015 with the institute DSMZ -Germany, and *Lactobacillus kefiri* LKF02 DSM 32080, deposited by Probiotical SpA on 10.07.2015 with the institute DSMZ -Germany for use in a method for the preventive or curative treatment of inflammatory intestinal diseases selected from the group comprising or, alternatively, consisting of: irritable bowel syndrome (IBS), inflammatory bowel disease (IBD), Crohn's disease, colics, colics in infants, colitis, and ulcerative colitis (UC).

2. The bacterial strains for use according to claim 1, wherein said strains are administered to adult subjects, preferably also with said disease in a chronic stage.

3. The bacterial strains for use according to claim 1, wherein said bacterial strains are for use in pediatrics.

4. A food composition, or a composition for a supplement, or a composition for a medical device, or a pharmaceutical composition comprising a mixture that comprises or, alternatively, consists of a bacterial strain selected from the group consisting of the bacterial strains belonging to the species *Lactobacillus kefiri,* according to claim 1 for use in the preventive or curative treatment of inflammatory intestinal diseases selected from the group comprising or, alternatively, consisting of: irritable bowel syndrome (IBS), inflammatory bowel disease (IBD), Crohn's disease, colics, colics in infants, colitis, and ulcerative colitis (UC), and optionally also contains excipients and/or substances of a food or pharmaceutical grade formulation.

5. A composition comprising a vegetable oil and a mixture of bacterial strains belonging to the species Lactobacillus kefiri selected from the group comprising or, alternatively, consisting of *Lactobacillus kefiri* LKF01 DSM 32079 and *Lactobacillus kefiri* LKF02 DSM 32080 for use in the preventive or curative treatment of inflammatory intestinal diseases selected from the group comprising or, alternatively, consisting of: irritable bowel syndrome (IBS), inflammatory bowel disease (IBD), Crohn's disease, colics, colics in infants, colitis, and ulcerative colitis (UC), said composition can optionally also contain excipients and/or substances of a food or pharmaceutical grade formulation.

6. The composition for use according to claim 5, wherein said composition comprises a vegetable oil selected from the group comprising or, alternatively, consisting of olive oil, soybean oil, corn oil, rice oil or sunflower oil.

7. The composition for use according to claim 5 or 6,
wherein said bacterial strains have the bacterial cells in coated or microencapsulated form with a coating material comprising or, alternatively, consisting of at least one lipid of animal or vegetable origin selected from the group comprising or, alternatively, consisting of:
(i) Glyceryl dipalmitostearate E471, INCI (PCPC): glyceryl distearate, CAS: 85251-77-0 (or 1323-83-7), EINECS: 286-490-9 (or 215-359-0); preferably Biogapress Vegetal BM 297 ATO -Gattefosse SAS;
(ii) Polyglyceryl palmitostearate E475, INCI: polyglyceryl-6-distearate, CAS: 61725-93-7; preferably Plurol Stearique WL 1009 -Gattefosse SAS, and
wherein said bacterial strains, preferably in lyophilised form or in granules or powder, are present in said composition at a concentration comprised from 1x10⁸ CFU/g to 1x10¹² CFU/g of composition, preferably at a concentration comprised from 1x10⁹ CFU/g to 1x10¹¹ CFU/g of composition.

8. The composition for use according to any one of claims 5-7, wherein said composition is for use in pediatrics in the preventive or curative treatment of inflammatory intestinal diseases selected form the group comprising or, alternatively, consisting of: irritable bowel syndrome (IBS), inflammatory bowel disease (IBS), Crohn's disease, colics, colics in infants, colitis, and ulcerative colitis (UC).

## Patentansprüche

1. Bakterienstämme der Gattung *Lactobacillus kefiri,* identifiziert als *Lactobacillus kefiri* LKF01 DSM 32079, hinterlegt von Probiotical SpA am 10.07.2015 beim Institut DSMZ-Deutschland, und *Lactobacillus kefiri* LKF02 DSM 32080, hinterlegt von Probiotical SpA am 10.07.2015 beim Institut DSMZ-Deutschland zur Verwendung in einem Verfahren zur präventiven oder kurativen Behandlung von entzündlichen Darmerkrankungen, ausgewählt aus der Gruppe, umfassend oder alternativ bestehend aus: Reizdarmsyndrom (IBS), entzündliche/r Darmerkrankung (IBD), Morbus Crohn, Koliken, Koliken bei Säuglingen, Kolitis und Colitis ulcerosa (UC).

2. Bakterienstämme zur Verwendung nach Anspruch 1, wobei die Stämme an erwachsene Probanden verabreicht werden, vorzugsweise auch mit der Krankheit in einem chronischen Stadium.

3. Bakterienstämme zur Verwendung nach Anspruch 1, wobei die Bakterienstämme zur Verwendung in der Pädiatrie bestimmt sind.

4. Nahrungsmittelzusammensetzung oder eine Zusammensetzung für ein Nahrungsergänzungsmittel oder eine Zusammensetzung für ein medizinisches Gerät oder eine pharmazeutische Zusammensetzung, die eine Mischung umfasst, die einen Bakterienstamm umfasst oder alternativ daraus besteht, der aus der Gruppe ausgewählt ist, bestehend aus den Bakterienstämmen der Gattung *Lactobacillus kefiri* nach Anspruch 1 zur Verwendung bei der präventiven oder kurativen Behandlung von entzündlichen Darmerkrankungen, ausgewählt aus der Gruppe, umfassend oder alternativ bestehend aus: Reizdarmsyndrom (IBS), entzündliche/r Darmerkrankung (IBD), Morbus Crohn, Koliken, Koliken bei Säuglingen, Kolitis und Colitis ulcerosa (UC), und gegebenenfalls auch Hilfsstoffeln und/oder Substanzen einer Formulierung in Lebensmittel- oder pharmazeutischer Qualität enthält.

5. Zusammensetzung, umfassend ein Pflanzenöl und eine Mischung von Bakterienstämmen der Gattung Lactobacillus kefiri, ausgewählt aus der Gruppe, umfassend oder alternativ bestehend aus *Lactobacillus kefiri* LKF01 DSM 32079 und *Lactobacillus kefiri* LKF02 DSM 32080 zur Verwendung bei der präventiven oder kurativen Behandlung von entzündlichen Darmerkrankungen, ausgewählt aus der Gruppe, umfassend oder alternativ bestehend aus: Reizdarmsyndrom (IBS), entzündliche/r Darmerkrankung (IBD), Morbus Crohn, Koliken, Koliken bei Säuglingen, Kolitis und Colitis ulcerosa (UC), wobei die Zusammensetzung optional auch Hilfsstoffe und/oder Substanzen einer Formulierung in Lebensmittel- oder pharmazeutischer Qualität enthalten kann.

6. Zusammensetzung zur Verwendung nach Anspruch 5, wobei die Zusammensetzung ein Pflanzenöl umfasst, das aus der Gruppe ausgewählt ist, umfassend oder alternativ bestehend aus Olivenöl, Sojaöl, Maisöl, Reisöl oder Sonnenblumenöl.

7. Zusammensetzung zur Verwendung nach Anspruch 5 oder 6,
wobei die Bakterienstämme die Bakterienzellen in beschichteter oder mikroverkapselter Form mit einem Beschichtungsmaterial aufweisen, umfassend oder alternativ bestehend aus mindestens einem Lipid tierischen oder pflanzlichen Ursprungs, ausgewählt aus der Gruppe, umfassend oder alternativ bestehend aus:
(i) Glyceryldipalmitostearat E471, INCI (PCPC): Glyceryldistearat, CAS: 85251-77-0 (oder 1323-83-7), EINECS: 286-490-9 (oder 215-359-0); vorzugsweise Biogapress Vegetal BM 297 ATO -Gattefosse SAS;
(ii) Polyglycerylpalmitostearat E475, INCI: Polyglyceryl-6-distearat, CAS: 61725-93-7; vorzugsweise Plurol Stearique WL 1009 -Gattefosse SAS, und
wobei die Bakterienstämme, vorzugsweise in lyophilisierter Form oder in Granulat oder Pulver, in der Zusammensetzung in einer Konzentration von 1x10⁸ KBE/g bis 1x10¹² KBE/g der Zusammensetzung, vorzugsweise in einer Konzentration von 1x10⁹ KBE/g bis 1x10¹¹ KBE/g der Zusammensetzung vorliegen.

8. Zusammensetzung zur Verwendung nach einem der Ansprüche 5-7, wobei die Zusammensetzung zur Verwendung in der Pädiatrie bei der präventiven oder kurativen Behandlung von entzündlichen Darmerkrankungen ist, ausgewählt aus der Gruppe, umfassend oder alternativ bestehend aus: Reizdarmsyndrom (IBS), entzündliche/r Darmerkrankung (IBS), Morbus Crohn, Koliken, Koliken bei Säuglingen, Colitis und Colitis ulcerosa (UC).

## Revendications

1. Souches bactériennes appartenant à l'espèce Lactobacillus kefiri, identifiées comme *Lactobacillus kefiri* LKF01 DSM 32079, déposées par Probiotical SpA le 10 juillet 2015 auprès de l'institut DSMZ -Allemagne, et *Lactobacillus kefiri* LKF02 DSM 32080, déposées par Probiotical SpA le 10 juillet 2015 auprès de l'institut DSMZ -Allemagne, destinées à être utilisées dans une méthode de traitement préventif ou curatif de maladies inflammatoires de l'intestin choisies dans le groupe comprenant ou, en variante, constitué par : le syndrome de l'intestin irritable (SII), maladies inflammatoires chroniques de l'intestin (MICI), la maladie de Crohn, les coliques, les coliques du nourrisson, la colite et la colite ulcéreuse (CU).

2. Souches bactériennes destinées à être utilisées selon la revendication 1, dans lesquelles lesdites souches sont administrées à des sujets adultes, de préférence également atteints de ladite maladie à un stade chronique.

3. Souches bactériennes destinées à être utilisées selon la revendication 1, dans lesquelles lesdites souches bactériennes sont destinées à être utilisées en pédiatrie.

4. Composition alimentaire, ou composition destinée à un complément alimentaire, ou composition destinée à un dispositif médical, ou composition pharmaceutique comprenant un mélange qui contient ou, en variante, est constitué d'une souche bactérienne choisie dans le groupe constitué par des souches bactériennes appartenant à l'espèce *Lactobacillus kefiri,* selon la revendication 1, destinée à être utilisée dans le traitement préventif ou curatif de maladies inflammatoires de l'intestin choisies dans le groupe comprenant ou, en variante, constitué par : le syndrome de l'intestin irritable (SII), maladies inflammatoires chroniques de l'intestin (MICI), la maladie de Crohn, les coliques, les coliques du nourrisson, la colite et la colite ulcéreuse (CU), et contenant éventuellement également des excipients et/ou des substances d'une formulation de qualité alimentaire ou pharmaceutique.

5. Composition comprenant une huile végétale et un mélange de souches bactériennes appartenant à l'espèce Lactobacillus kefiri choisies dans le groupe comprenant ou, en variante, constitué par *Lactobacillus kefiri* LKF01 DSM 32079 et *Lactobacillus kefiri* LKF02 DSM 32080, destinée à être utilisée dans le traitement préventif ou curatif de maladies inflammatoires de l'intestin choisies dans le groupe comprenant ou, en variante, constitué par : le syndrome de l'intestin irritable (SII), maladies inflammatoires chroniques de l'intestin (MICI), la maladie de Crohn, les coliques, les coliques du nourrisson, la colite et la colite ulcéreuse (CU), ladite composition pouvant éventuellement contenir également des excipients et/ou des substances de qualité alimentaire ou pharmaceutique.

6. Composition destinée à être utilisée selon la revendication 5, dans laquelle ladite composition comprend une huile végétale choisie dans le groupe comprenant ou, en variante, constitué par : huile d'olive, huile de soja, huile de maïs, huile de riz ou huile de tournesol.

7. Composition destinée à être utilisée selon la revendication 5 ou 6,
dans laquelle lesdites souches bactériennes présentent des cellules bactériennes sous forme enrobée ou microencapsulée avec un matériau d'enrobage comprenant ou, en variante, constitué d'au moins un lipide d'origine animale ou végétale choisi dans le groupe comprenant ou, en variante, constitué par :
(i) dipalmitostéarate de glycéryle E471, INCI (PCPC) : distéarate de glycéryle, CAS : 85251-77-0 (ou 1323-83-7), EINECS : 286-490-9 (ou 215-359-0) ; de préférence Biogapress Vegetal BM 297 ATO - Gattefosse SAS ;
(ii) palmitostéarate de polyglycéryle E475, INCI : distéarate de polyglycéryle-6, CAS : 61725-93-7 ; de préférence Plurol Stéarique WL 1009 -Gattefosse SAS, et
dans laquelle lesdites souches bactériennes, de préférence sous forme lyophilisée ou en granulés ou en poudre, sont présentes dans ladite composition à une concentration comprise de 1x10⁸ à 1x10¹² UFC/g de composition, de préférence à une concentration comprise de 1x10⁹ à 1x10¹¹ UFC/g de composition.

8. Composition destinée à être utilisée selon l'une quelconque des revendications 5-7, dans laquelle ladite composition est destinée à être utilisée en pédiatrie dans le traitement préventif ou curatif de maladies inflammatoires de l'intestin choisies dans le groupe comprenant ou, en variante constitué par : le syndrome de l'intestin irritable (SII), maladies inflammatoires chroniques de l'intestin (MICI), la maladie de Crohn, les coliques, les coliques du nourrisson, la colite et la colite ulcéreuse (CU).
